(19) Europäisches Patentamt
European Patent Office
Office européen des brevets

(11) Veröffentlichungsnummer: **0 045 525 B1**

# (12) EUROPÄISCHE PATENTSCHRIFT

(45) Veröffentlichungstag der Patentschrift: **25.04.84**

(51) Int. Cl.³: **C 07 D 501/36,** A 61 K 31/545

(21) Anmeldenummer: **81106777.6**

(22) Anmeldetag: **30.05.79**

(60) Veröffentlichungsnummer der früheren Anmeldung nach Art. 76 EPÜ:

(54) **Cephalosporinderivate und deren Herstellung.**

(30) Priorität: **30.05.78 CH 5882/78**
**08.03.79 CH 2248/79**

(43) Veröffentlichungstag der Anmeldung: **10.02.82 Patentblatt 82/6**

(45) Bekanntmachung des Hinweises auf die Patenterteilung: **25.04.84 Patentblatt 84/17**

(84) Benannte Vertragsstaaten: **AT BE CH DE FR IT LU NL SE**

(56) Entgegenhaltungen:
**EP - A - 0 000 005**
**DE - A - 2 707 565**
**FR - A - 2 275 215**
**FR - A - 2 280 381**
**FR - A - 2 348 218**
**LU - A - 80 669**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE & CO. Aktiengesellschaft, CH-4002 Basel (CH)**

(72) Erfinder: **Montavon, Marc, Dr., Realpstrasse 72, CH-4054 Basel (CH)**
Erfinder: **Reiner, Roland, Dr., Rheinfelderstrasse 8, CH-4058 Basel (CH)**

(74) Vertreter: **Lederer, Franz, Dr. et al, Patentanwälte Dr. Franz Lederer Reiner F. Meyer Lucile-Grahn-Strasse 22, D-8000 München 80 (DE)**

EP 0 045 525 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Cephalosporinderivate und deren Herstellung

Die vorliegende Erfindung betrifft neue Cephalosporinderivate der allgemeinen Formel

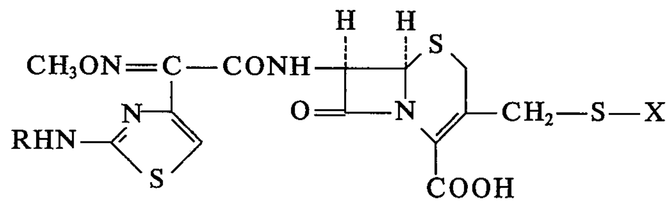

(II)

in der X die 1,2,5,6-Tetrahydro-2-methyl-5,6-dioxoas-triazin-3-ylgruppe oder deren entsprechende tautomere Form, bzw. die 2,5-Dihydro-6-hydroxy-2-methyl-5-oxo-astriazin-3-ylgruppe bedeutet, R eine abspaltbare Schutzgruppe ausgewählt zwischen sauer-hydrolytisch abspaltbaren Schutzgruppen, basisch-hydrolytisch abspaltbaren Schutzgruppen und Chlor-, Brom- und Jodacetyl darstellt und die Carboxygruppe in geschützter Form vorliegen kann.

Die Cephalosporinderivate der Formel II sind wertvolle Zwischenprodukte für die Herstellung von pharmakologisch wertvollen Cephalosporinderivaten. Sie können durch Abspaltung der Schutzgruppe R, gegebenenfalls auch die allenfalls vorhandene Carboxyschutzgruppe, in das pharmakologisch wertvolle Cephalosporinderivat der allgemeinen Formel

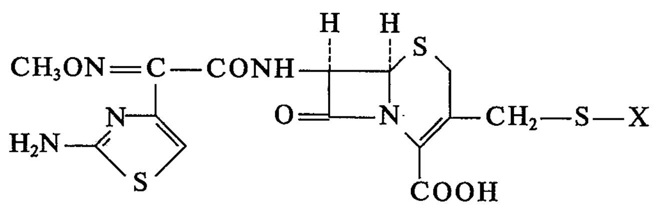

(I)

in der X die obige Bedeutung hat sowie in seine leicht hydrolysierbaren Ester/Äther und Salze dieser Verbindung und Hydrate der Verbindung der Formel I bzw. von deren Estern/Äthern und Salzen übergeführt werden.

Die erfindungsgemäßen Produkte können in der synisomeren Form

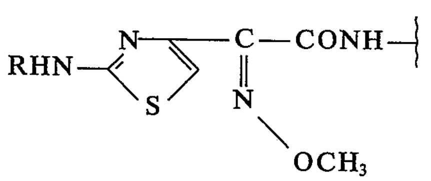

oder in der anti-isomeren Form

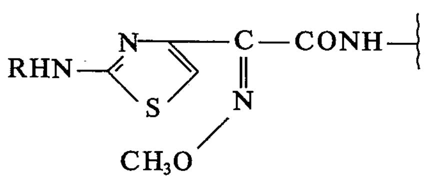

bzw. als Gemische dieser beiden Formen vorliegen. Bevorzugt ist die syn-isomere Form bzw. Gemische, in denen die syn-isomere Form überwiegt.

Ein bevorzugtes Zwischenprodukt ist die (6R,7R)-7-/2-[2-(2-Chloracetamido)-4-thiazolyl]-2-(Z-methoxyimino)acetamido/-3-/[(2,5-dihydro-6-hydroxy-2-methyl-5-oxo-as-triazin-3-yl)-thio]methyl/-8-oxo-5-thia-1-azabicyclo[4.2.0]oct-2-en-2-carbonsäure.

Die im erfindungsgemäßen Zwischenprodukt der Formel II vorhandene Carboxygruppe kann wahlweise geschützt sein, z. B. durch Veresterung zu einem leicht spaltbaren Ester, wie dem Silylester, z. B. dem Trimethylsilylester. Es kommen auch die oben erläuterten, leicht hydrolysierbaren Ester in Betracht. Die Carboxygruppe kann auch durch Salzbildung mit einer anorganischen oder tertiären organischen Base, wie Triäthylamin, geschützt werden. Die R-Schutzgruppen sind entweder sauer-hydrolytisch abspaltbare Schutzgruppen, wie z. B. t-Butoxycarbonyl oder Trityl, basisch-hydrolytisch abspaltbare Schutzgruppen, wie z. B. Trifluoracetyl, oder auch, vorzugsweise Chlor-, Brom- und Jodacetyl, insbesondere Chloracetyl. Letztere Schutzgruppen können durch Behandeln mit

Thioharnstoff abgespalten werden.

Die erfindungsgemäßen Verbindungen der Formel II können z. B. durch N-Acylierung der entsprechenden 7-Aminoverbindung hergestellt werden, und zwar indem man eine Verbindung der allgemeinen Formel

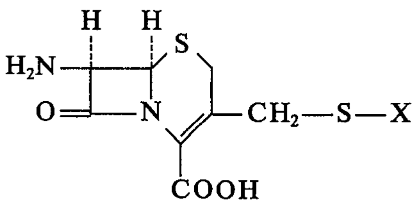

(III)

in der X die oben gegebene Bedeutung hat und die Carboxygruppe und/oder die Aminogruppe in geschützter Form vorliegen kann, mit einer Säure der allgemeinen Formel

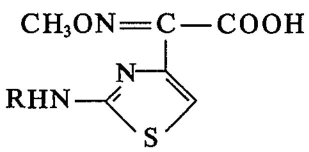

(IV)

in der R die oben gegebene Bedeutung hat, oder mit einem reaktionsfähigen funktionellen Derivat dieser Säure umsetzt und eine allenfalls vorhandene Carboxyschutzgruppe gegebenenfalls abspaltet.

Die in der 7-Aminoverbindung der Formel III vorhandene Carboxygruppe kann wahlweise geschützt sein, und zwar in der oben für die herzustellende Ausgangsverbindung der Formel II erläuterten Weise. Die Aminogruppe der Verbindung der Formel III kann z. B. durch eine Silylschutzgruppe, wie Trimethylsilyl, geschützt sein.

Als reaktionsfähige funktionelle Derivate von Säuren der Formel IV kommen z. B. Halogenide, d. h. Chloride, Bromide und Fluoride; Azide; Anhydride, insbesondere gemischte Anhydride mit stärkeren Säuren; reaktionsfähige Ester, z. B. N-Hydroxysuccinimidester, und Amide, z. B. Imidazolide, in Betracht.

Die Umsetzung der 7-Aminoverbindung der Formel III mit der Säure der Formel IV oder einem reaktionsfähigen funktionellen Derivat davon kann in an sich bekannter Weise durchgeführt werden. So kann man z. B. eine freie Säure der Formel IV mit einem der erwähnten Ester entsprechend Formel III mittels eines Carbodiimids, wie Dicyclohexylcarbodiimid, in einem inerten Lösungsmittel, wie Essigester, Acetonitril, Dioxan, Chloroform, Methylenchlorid, Benzol oder Dimethylformamid, kondensieren und anschließend die Estergruppe abspalten. Anstelle von Carbodiimiden lassen sich als Kondensationsmittel auch Oxazoliumsalze, z. B. N-Äthyl-5-phenyl-isoxazolium-3′-sulfonat, verwenden.

Nach einer anderen Ausführungsform setzt man ein Salz einer Säure der Formel III, z. B. ein Trialkylammoniumsalz, wie das Triäthylammoniumsalz, mit einem reaktionsfähigen funktionellen Derivat einer Säure der Formel IV wie oben erwähnt in einem inerten Lösungsmittel, z. B. einem der oben genannten, um.

Nach einer weiteren Ausführungsform wird ein Säurehalogenid, vorzugsweise das Chlorid einer Säure der Formel IV mit dem Amin der Formel III umgesetzt. Die Umsetzung erfolgt vorzugsweise in Gegenwart eines säurebindenden Mittels, z. B. in Gegenwart von wäßrigem Alkali, vorzugsweise Natronlauge, oder auch in Gegenwart eines Alkalimetallcarbonats, wie Kaliumcarbonat, oder in Gegenwart eines nieder-alkylierten Amins, wie Triäthylamin. Als Lösungsmittel wird vorzugsweise Wasser verwendet, gegebenenfalls im Gemisch mit einem inerten organischen Lösungsmittel, wie Tetrahydrofuran oder Dioxan. Es kann auch in einem aprotischen organischen Lösungsmittel, wie z. B. Dimethylformamid, Dimethylsulfoxid oder Hexamethylphosphorsäuretriamid, gearbeitet werden. Bei Verwendung von silylierten Ausgangsverbindungen der Formel III wird in wasserfreiem Medium gearbeitet.

Die Umsetzung der 7-Aminoverbindung der Formel III mit der Säure der Formel IV oder einem reaktionsfähigen funktionellen Derivat davon kann zweckmäßig bei Temperaturen zwischen etwa −40° C und Zimmertemperatur, beispielsweise bei etwa 0−10° C, erfolgen.

Die oben verwendeten 7-Aminoverbindungen der Formel III können ausgehend von einer Verbindung der Formel

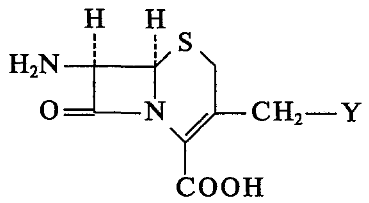

(V)

in der Y eine austretende Gruppe darstellt, durch Umsetzung mit einem Thiol der allgemeinen Formel

$$HS—X \qquad (VI)$$

in der X die oben gegebene Bedeutung hat, hergestellt werden.

Als austretende Gruppe Y einer Verbindung der Formel V kommen beispielsweise Halogene, z. B. Chlor, Brom oder Jod, Acyloxyreste, z. B. niedere Alkanoyloxyreste, wie Acetoxy, niedere Alkyl- oder Arylsulfonyloxyreste, wie Mesyloxy oder Tosyloxy, oder der Azidorest in Frage.

Die Umsetzung der Verbindung der Formel V mit dem Thiol der Formel VI kann in an sich bekannter Weise, z. B. bei einer Temperatur zwischen etwa 40 und 80°C, zweckmäßig bei etwa 60°C, in Wasser oder in einer Pufferlösung mit einem pH von etwa 6 bis 7, vorzugsweise 6,5, durchgeführt werden.

In der DE-A-2 715 385 und der DE-A-2 707 565 sind Cephalosporinderivate offenbart, welche, ähnlich wie die vorliegenden Cephalosporine der Formel I eine 7-[2-(2-Amino-4-thiazolyl)-2-(Z-methoxyimino)-acetamido]-gruppe tragen. Die vorliegenden Cephalosporine der Formel I unterscheiden sich jedoch charakteristisch durch die Konfiguration des Substituenten in 3-Stellung, wodurch neue Verbindungen mit überraschenden Wirkungsvorteilen zur Verfügung gestellt werden.

Die erfindungsgemäßen Zwischenprodukte erfüllen die Aufgabe, neue und bessere Cephalosporinderivate zur Verfügung zu stellen, indem sie, wie gesagt, durch Abspaltung der Schutzgruppe R, gegebenenfalls auch die allenfalls vorhandene Carboxyschutzgruppe, in das pharmakologisch wertvolle Cephalosporinderivat der allgemeinen Formel I übergeführt werden können.

## Beispiel

22,24 g 2-(2-Chloracetamido-thiazol-4-yl)-2-(Z-methoxyimino)-essigsäure werden in 240 ml Methylenchlorid suspendiert. Dieser Suspension werden 13,39 ml Triäthylamin zugegeben, wobei eine hellbraune Lösung entsteht. Diese Lösung wird auf 0—5°C gekühlt und mit 16,72 g Phosphorpentachlorid versetzt, 5 Minuten bei 0—5°C und 20 Minuten ohne Kühlung gerührt. Die gelbe Lösung wird am Vakuum bei 35°C eingedampft. Der Eindampfrückstand wird zweimal mit n-Heptan geschüttelt und letzteres abdekantiert. Der harzige Rückstand wird mit 240 ml Tetrahydrofuran behandelt und das ungelöste Triäthylamin-hydrochlorid wird abfiltriert. Das gelbe Filtrat enthält das Säurechlorid.

22 g (7R)-7-Amino-3-desacetoxy-3-[(2,5-dihydro-6-hydroxy-2-methyl-5-oxo-as-triazin-3-yl)-thio]-cephalosporansäure werden in einem Gemisch von 300 ml Wasser und 150 ml Tetrahydrofuran suspendiert. Zur Suspension wird unter guter Stickstoff-Begasung mit Hilfe des Autotitrators 2-n Natronlauge zugetropft, bis eine braunrote Lösung von pH 8 entsteht. Diese wird auf 0—5°C gekühlt und während 15 Minuten tropfenweise mit der oben hergestellten Lösung des Säurechlorids in Tetrahydrofuran versetzt. Danach wird 2½ Stunden bei 25°C gerührt. Das pH des Acylierungsgemisches wird unter Zugabe von 2-n Natronlauge konstant bei 8 gehalten. Die praktisch schwarze Lösung wird am Vakuum bei 40°C von Tetrahydrofuran befreit. Nun werden 100 ml 2-n Schwefelsäure zugegeben. Die dabei ausgefallene Substanz wird abgenutscht, mit Wasser gewaschen und gut abgesaugt. Das feuchte, braune Nutschgut wird in 1,5 l Aceton gelöst. Die dunkle Lösung wird von wenig dunklem ungelöstem Material über Hyflo abfiltriert, mit Kohle versetzt, 30 Minuten gerührt und wieder über Hyflo filtriert. Das orange-rote Filtrat wird über Natriumsulfat getrocknet, am Vakuum eingeengt und mit Essigester abgedampft. Dabei fällt ein schwarzes Harz aus, das abfiltriert und verworfen wird.

Das 2-phasige, noch Wasser enthaltende Filtrat wird dreimal mit Benzol am Vakuum bei 40°C azeotropiert. Die dabei ausgefallene Substanz wird abgenutscht und im Vakuum bei 40°C getrocknet. Letztere wird zweimal mit je 1 l Aceton verrührt, wobei ein braunes Harz zurückbleibt, das verworfen wird. Die vereinigten orangefarbigen Acetonextrakte werden am Vakuum bei 40°C auf ca. 150 ml eingeengt, wobei ein braunes Harz abfiltriert und verworfen wird. Das Filtrat wird mit 1 Liter Essigester versetzt und am Vakuum bei 40°C eingeengt. Die dabei ausgefallene Substanz wird abgenutscht, mit Essigester und danach mit Äther gewaschen [(6R,7R)-7-/2-[2-(2-Chloracetamido)-4-thiazolyl]-2-(Z-methoxyimino)acetamido/-3-/[(2,5-dihydro-6-hydroxy-2-methyl-5-oxo-as-triazin-3-yl)thio]-methyl-8-oxo-5-thia-1-azabicyclo[4.2.0]oct-2-en-2-carbonsäure, Fraktion 1: eine beige, amorphe Säure].

Die Essigestermutterlauge wird am Vakuum bei 40°C stark eingeengt, mit Äther verdünnt und die ausgefallene Substanz abgenutscht [(6R,7R)-7-/2-[2-(2-Chloracetamido)-4-thiazolyl]-2-(Z-methoxyimino)acetamido/-3-/[(2,5-dihydro-6-hydroxy-2-methyl-5-oxo-as-triazin-3-yl)thio]-methyl/-8-oxo-5-thia-1-azabicyclo[4.2.0]oct-2-en-2-carbonsäure, Fraktion II: eine hellbeige amorphe Säure, dünnschichtchromatographisch etwas reiner als Fraktion I].

Zur Herstellung des Dinatriumsalzes werden 3,5 g Säure (Fraktion II) in einem Gemisch von 20 ml Aceton und 11 ml Wasser gelöst. Die Lösung wird mit 7 ml einer 2-n Lösung von 2-Äthylcapronsäure-Natriumsalz in Essigester versetzt, wobei das Dinatriumsalz kristallisiert. Nun werden noch portionenweise 25 ml Aceton hinzugefügt und das Gemisch 2 Stunden im Tiefkühlschrank aufbewahrt. Danach wird das Kristallisat abgenutscht, nacheinander mit 25 ml eines eiskalten Aceton-Wasser-Gemisches (80 : 20), reinem Aceton und tiefsiedendem Petroläther gewaschen und über Nacht im Hochvakuum bei 40° C getrocknet. Man erhält das Dinatriumsalz der (6R,7R)-7-/2-[2-(2-Chloracetamido)-4-thiazolyl]-2-(Z-methoxyimino)-acetamido/-3-/[(2,5-dihydro-6-hydroxy-2-methyl-5-oxo-as-triazin-3-yl)thio]-methyl/-8-oxo-5-thia-1-azabicyclo[4.2.0]-oct-2-en-2-carbonsäure als hellgelbe Kristalle. $[\alpha]_D^{20} = -142{,}7°$ (c=1 in Wasser). Das Kernresonanzspektrum und die Mikroanalyse entsprechen der angegebenen Struktur.

**Patentansprüche für die Vertragsstaaten: BE, CH, DE, FR, IT, LU, NL, SE**

1. Cephalosporinderivate der allgemeinen Formel

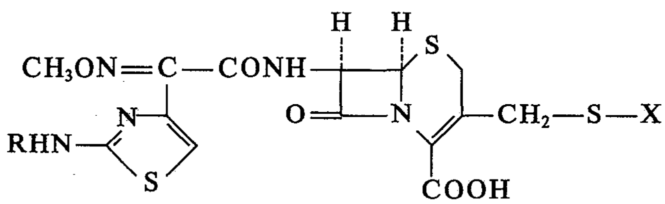

(II)

in der X die 1,2,5,6-Tetrahydro-2-methyl-5,6-dioxo-as-triazin-3-ylgruppe bzw. die 2,5-Dihydro-6-hydroxy-2-methyl-5-oxo-as-triazin-3-ylgruppe bedeutet, R eine abspaltbare Schutzgruppe ausgewählt zwischen sauer-hydrolytisch abspaltbaren Schutzgruppen, basisch-hydrolytisch abspaltbaren Schutzgruppen und Chlor-, Brom- und Jodacetyl darstellt und die Carboxygruppe in geschützter Form vorliegen kann.

2. Cephalosporinderivate gemäß Anspruch 1 in der syn-isomeren Form bzw. Gemische, in denen die syn-isomere Form überwiegt.

3. (6R,7R)-7-/2-[2-(2-Chloracetamido)-4-thiazolyl]-2-(Z-methoxyimino)acetamido/-3-/[(2,5-dihydro-6-hydroxy-2-methyl-5-oxo-as-triazin-3-yl)-thio]methyl/-8-oxo-5-thia-1-azabicyclo[4.2.0]oct-2-en-2-carbonsäure.

4. Verfahren zur Herstellung der Verbindungen gemäß einem der Ansprüche 1–3, dadurch gekennzeichnet, daß man eine Verbindung der allgemeinen Formel

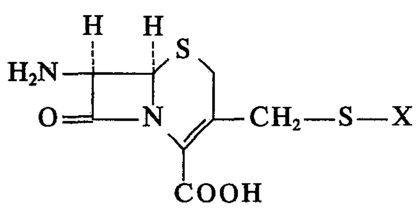

(III)

in der X die in Anspruch 1 gegebene Bedeutung hat und die Carboxygruppe und/oder die Aminogruppe in geschützter Form vorliegen kann, mit einer Säure der allgemeinen Formel

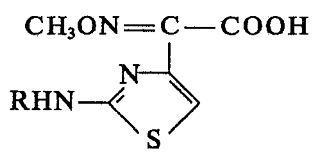

(IV)

in der R die in Anspruch 1 gegebene Bedeutung hat, oder mit einem reaktionsfähigen funktionellen Derivat dieser Säure umsetzt und eine allenfalls vorhandene Carboxyschutzgruppe gegebenenfalls abspaltet.

**Patentansprüche für den Vertragsstaat: AT**

1. Verfahren zur Herstellung von Cephalosporinderivaten der allgemeinen Formel

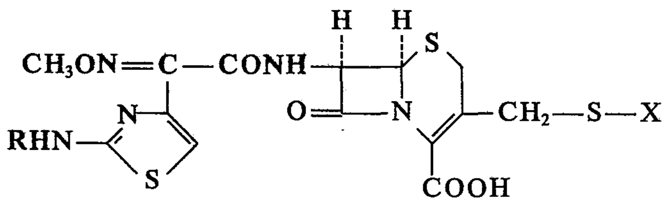

(II)

in der X die 1,2,5,6-Tetrahydro-2-methyl-5,6-dioxo-as-triazin-3-ylgruppe bzw. die 2,5-Dihydro-6-hydroxy-2-methyl-5-oxo-as-triazin-3-ylgruppe bedeutet, R eine abspaltbare Schutzgruppe ausgewählt zwischen sauer-hydrolytisch abspaltbaren Schutzgruppen, basisch-hydrolytisch abspaltbaren Schutzgruppen und Chlor-, Brom- und Jodacetyl darstellt und die Carboxygruppe in geschützter Form vorliegen kann, dadurch gekennzeichnet, daß man eine Verbindung der allgemeinen Formel

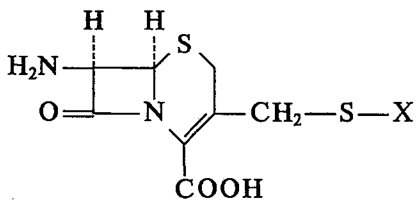

(III)

in der X die oben gegebene Bedeutung hat und die Carboxygruppe und/oder die Aminogruppe in geschützter Form vorliegen kann, mit einer Säure der allgemeinen Formel

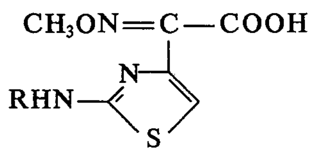

(IV)

in der R die oben gegebene Bedeutung hat, oder mit einem reaktionsfähigen funktionellen Derivat dieser Säure umsetzt und eine allenfalls vorhandene Carboxyschutzgruppe gegebenenfalls abspaltet.

2. Verfahren nach Anspruch 1 zur Herstellung von Cephalosporinderivaten in der syn-isomeren Form bzw. von Gemischen, in denen die syn-isomere Form überwiegt, dadurch gekennzeichnet, daß man ein erhaltenes syn/anti-Gemisch in üblicher Weise auftrennt.

3. Verfahren nach Anspruch 2 zur Herstellung von (6R,7R)-7-/2-[2-(2-Chloracetamido)-4-thiazolyl]-2-(Z-methoxyimino)acetamido/-3-/[(2,5-dihydro-6-hydroxy-2-methyl-5-oxo-as-triazin-3-yl)-thio]methyl/-8-oxo-5-thia-1-azabicyclo[4.2.0]oct-2-en-2-carbonsäure, dadurch gekennzeichnet, daß man entsprechend substituierte Ausgangsverbindungen einsetzt.

**Patent Claims for the Contracting States: BE, CH, DE, FR, IT, LU, NL, SE**

1. Cephalosporin derivatives of the general formula

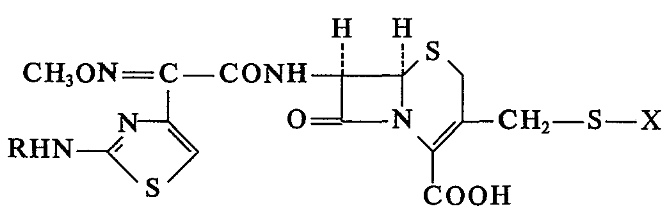

(II)

in which X signifies the 1,2,5,6-tetrahydro-2-methyl-5,6-dioxo-as-triazin-3-yl group or the 2,5-dihydro-6-hydroxy-2-methyl-5-oxo-as-triazin-3-yl group, R represents a cleavable protecting group selected from protecting groups cleavable by acid hydrolysis, protecting groups cleavable by basic hydrolysis and chloroacetyl, bromoacetyl and iodoacetyl and the carboxy group can be present in protected form.

2. Cephalosporin derivatives according to claim 1 in the syn-isomeric form or as mixtures in which the synisomeric form predominates.

3. (6R,7R)-7-/2-[2-(2-Chloroacetamido)-4-thiazolyl]-2-(Z-methoxyimino)acetamido/-3-/[(2,5-dihydro-6-hydroxy-2-methyl-5-oxo-as-triazin-3-yl)-thio]methyl/-8-oxo-5-thia-1-azabicyclo[4.2.0]oct-2-ene-2-carboxylic acid.

4. A process for the manufacture of the compounds according to any one of claims 1–3, characterized by reacting a compound of the general formula

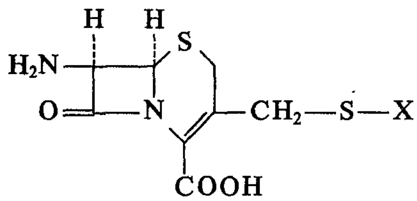

(III)

in which X has the significance given in claim 1 and the carboxy group and/or the amino group can be present in protected form, with an acid of the general formula

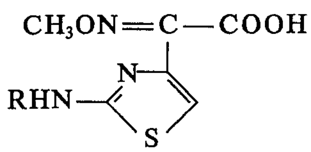

(IV)

in which R has the significance given in claim 1, or with a reactive functional derivative of this acid and, if desired, cleaving off a carboxy protecting group which may be present.

**Patent Claims for the Contracting State: AT**

1. A process for the manufacture of cephalosporin derivatives of the general formula

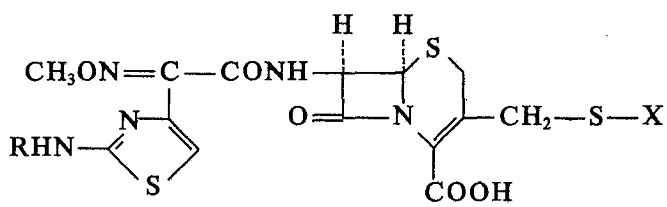

(II)

in which X signifies the 1,2,5,6-tetrahydro-2-methyl-5,6-dioxo-as-triazin-3-yl group or the 2,5-dihydro-6-hydroxy-2-methyl-5-oxo-as-triazin-3-yl group, R represents a cleavable protecting group selected from protecting groups cleavable by acid hydrolysis, protecting groups cleavable by basic hydrolysis and chloroacetyl, bromoacetyl and iodoacetyl and the carboxy group can be present in protected form, characterized by reacting a compound of the general formula

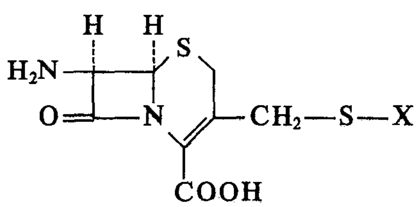

(III)

in which X has the significance given above and the carboxy group and/or the amino group can be present in protected form, with an acid of the general formula

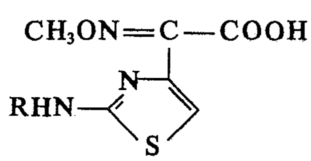

(IV)

in which R has the significance given above, or with a reactive functional derivative of this acid and, if desired, cleaving off a carboxy protecting group which may be present.

2. A process according to claim 1 for the manufacture of cephalosporin derivatives in the

syn-isomeric form or of mixtures in which the syn-isomeric form predominates, characterized by separating in the usual manner a syn/anti mixture obtained.

3. A process according to claim 2 for the manufacture of (6R,7R)-7-/2-[2-(2-chloroacetamido)-4-thiazolyl]-2-(Z-methoxyimino)acetamido/-3-/[(2,5-dihydro-6-hydroxy-2-methyl-5-oxo-as-triazin-3-yl)-thio]methyl/-8-oxo-5-thia-1-azabicyclo[4.2.0]oct-2-ene-2-carboxylic acid, characterized by using correspondingly substituted starting compounds.

**Revendications pour les Etats contractants: BE, CH, OE, FR, IT, LU, NL, SE**

1. Dérivés de céphalosporine de formule générale

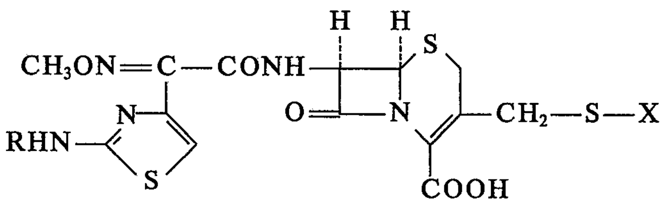

(II)

où X représente le groupe 1,2,5,6-tétrahydro-2-méthyl-5,6-dioxo-as-triazin-3-yle ou le groupe 2,5-dihydro-6-hydroxy-2-méthyl-5-oxo-as-triazin-3-yle, R représente un groupe protecteur séparable choisi entre les groupes protecteurs séparables par hydrolyse acide, les groupes protecteurs séparable par hydrolyse basique et le chloro-, le bromo- et le iodo-acétyle, et le groupe carboxy peut se présenter sous forme protégée.

2. Dérivés de céphalosporine selon la revendication 1 sous la forme isomère syn ou mélanges où la forme isomère syn prédomine.

3. Acide (6R,7R)-7-/2-[2-(2-chloracétamido)-4-thiazolyl]-2-(Z-méthoxyimino)acétamido/-3-/[(2,5-dihydro-6-hydroxy-2-méthyl-5-oxo-as-triazin-3-yl)-thio]méthyl/-8-oxo-5-thia-1-azabicyclo[4.2.0]oct-2-ène-2-carboxylique.

4. Procédé de préparation des composés selon l'une des revendications 1—3, caractérisé en ce qu'on fait réagir un composé de formule générale

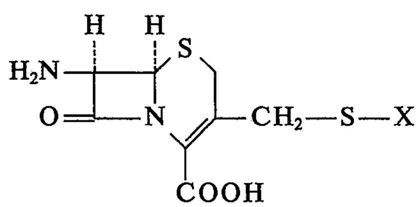

(III)

où X a la signification donnée dans la revendication 1 et le groupe carboxy et/ou le groupe amino peut se présenter sous forme protégée, avec un acide de formule générale

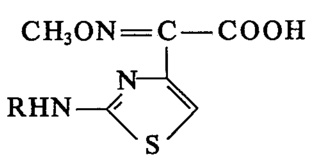

(IV)

où R a la signification donnée dans la revendication 1 ou avec un dérivé fonctionnel réactif de cet acide, et ce qu'on sépare le cas échéant un groupe protecteur de carboxy éventuellement présent.

**Revendications pour l'Etat contractant: AT**

1. Procédé de préparation de dérivés de céphalosporine de formule générale

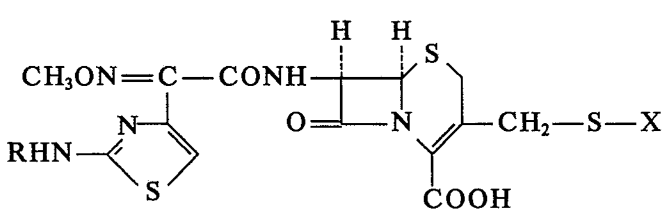

(II)

où X représente le groupe 1,2,5,6-tetrahydro-2-méthyl-5,6-dioxo-as-triazin-3-yle ou le groupe 2,5-dihydro-6-hydroxy-2-méthyl-5-oxo-as-triazin-3-yle, R représente un groupe protecteur séparable choisi entre les groupes protecteurs séparables par hydrolyse acide, les groupes protecteurs séparables par hydrolyse basique et le chloro-, le bromo- et l'iodo-acétyle, et le groupe carboxy peut se présenter sous forme protégée, caractérisé en ce qu'on fait réagir un composé de formule générale

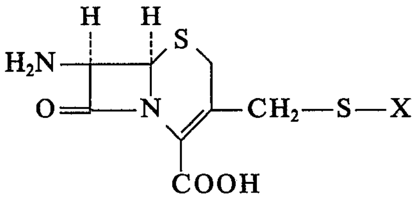

(III)

où X a la signification donnée ci-dessus et le groupe carboxy et/ou le groupe amino peut se présenter sous forme protégée, avec un acide de formule générale

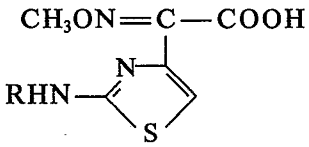

(IV)

où R a la signification donnée ci-dessus, ou avec un dérivé fonctionnel réactif de cet acide, et en ce qu'on sépare le cas échéant un groupe protecteur de carboxy éventuellement présent.

2. Procédé selon la revendication 1 de préparation de dérivés de céphalosporine sous la forme isomère syn ou de mélanges où la forme isomère syn prédomine, caractérisé en ce qu'on sépare de manière habituelle un mélange syn/anti obtenu.

3. Procédé selon la revendication 2 de préparation de l'acide (6R,7R)-7-/2-[2-(2-chloracétamido)-4-thiazolyl]-2-(Z-méthoxyimino)acétamido/-3-/[(2,5-dihydro-6-hydroxy-2-méthyl-5-oxo-as-triazin-3-yl)-thio]méthyl/-8-oxo-5-thia-1-azabicyclo[4.2.0]oct-2-ène-2-carboxylique caractérisé en ce qu'on utilise des composés de départ substitués de façon correspondante.